# EUROPÄISCHE PATENTANMELDUNG

(11) **EP 2 105 161 A2**
(43) Veröffentlichungstag der Anmeldung: **30.09.2009**
(21) Anmeldenummer: 09154204.3
(22) Anmeldetag: 03.03.2009
(51) Int. Cl.: A61N 1/05

(54) **Signalleitung einer implantierbaren elektromedizinischen Anordnung**

(30) Priorität: 29.03.2008 DE 102008016364
(71) Anmelder: Biotronik CRM Patent AG, 6341 Baar (CH)
(72) Erfinder: Weiss, Ingo, 12435, Berlin (DE); Knorr, Stefan, 10119, Berlin (DE)
(74) Vertreter: Lindner-Vogt, Karin L.

(57) **Zusammenfassung**

Signalleitung einer elektrischen Anordnung, insbesondere einer implantierbaren elektromedizinischen Anordnung, mit einem elektrischen Leitungsabschnitt oder Leitungsende und einem mechanoelektrischen Wandler zur Wandlung eines elektrischen Wechselspannungssignals in eine mechanische Schwingung.

## Beschreibung

Die Erfindung betrifft eine Signalleitung einer elektrischen Anordnung, insbesondere einer implantierbaren elektromedizinischen Anordnung, zur funktionsmäßigen Verbindung eines Basis-Gerätes mit einem von diesem entfernten Sensor oder Aktor.

In den nachfolgenden Ausführungen wird weitgehend davon ausgegangen, dass sowohl das Basis-Gerät als auch der Sensor bzw. Aktor ebenfalls implantierbar und im Gebrauchszustand auch tatsächlich in den Körper eines Lebewesens, insbesondere eines Menschen oder anderen Säugetiers, implantiert sind. Grundsätzlich ist die Erfindung aber auch in andersartigen elektrischen Mess- bzw. Wirkanordnungen realisierbar.

Eine seit Jahrzehnten bekannte und mittlerweile die verbreitetste Anordnung der in Rede stehenden Art ist die Herzschrittmacher-Anordnung, die aus einem Herzschrittmacher als Basis-Gerät, mindestens einer an diesen angeschlossenen Elektrodenleitung als Signalleitung mit mindestens einer eine deren distalem Ende vorgesehenen und im Gebrauchszustand im oder am Herzen eines Patienten platzierten Elektrode besteht. Geräte dieser Art sind aber auch implantierbare Defibrillatoren, Stimulationsanordnungen zur Stimulation des Hörnervs oder implantierbare Mess- und Übertragungsanordnungen zur intrakorporalen Erfassung und Auswertung und/oder externen Übertragung von Messwerten physiologischer Größen. Es können in ein und derselben Anordnung neben dem Basis-Gerät sowohl Aktoren als auch Sensoren vorgesehen sein, wie beispielsweise bei Schrittmacheranordnungen mit Abfühlelektroden bzw. anderen Sensoren, etwa zur Erfassung der Blutsauerstoffsättigung oder des gefäßinternen Blutdrucks.

Bei derartigen Anordnungen ist die Signalleitung typischerweise eine elektrische Leitung, die mindestens einen langgestreckten, zumeist wendelförmig strukturierten oder auch in Seilform ausgestalteten elektrischen Leiter enthält. Bei Anordnungen mit mehreren Aktoren und/oder Sensoren ist das Basis-Gerät mit jedem Aktor bzw. Sensor über einen separaten und hinreichend isolierten Leiterdraht verbunden. Derartige Signalleitungen sind in großer Vielgestaltigkeit bekannt und haben sich seit Jahrzehnten in vielerlei Anwendungen bewährt. Dass sie insbesondere dann, wenn sie mehrere separate Leiter umfassen, relativ dick und starr sind und gelegentlich auch eine Störanfälligkeit gegenüber äußeren elektromagnetischen Feldern beobachtet wird, hat die Fachwelt offensichtlich akzeptiert.

Der Erfindung liegt die Aufgabe zugrunde, eine verbesserte Signalleitung der in Rede stehenden Art anzugeben, deren grundsätzlicher Aufbau insbesondere die Möglichkeit einer dünneren und weniger starren konstruktiven Ausführung und einer Erhöhung der Störsicherheit gegenüber äußeren Feldern in sich birgt.

Diese Aufgabe wird durch eine Signalleitung mit den Merkmalen des Anspruchs 1 gelöst. Zweckmäßige Fortbildungen des Erfindungsgedankens sind Gegenstand der abhängigen Ansprüche. Es wird des Weiteren eine implantierbare Elektrostimulationsanordnung mit einer derartigen Signalleitung vorgeschlagen.

Die Erfindung basiert auf dem Gedanken eines Abgehens vom Konzept der durchgängig elektrischen Signalübertragung zwischen dem Basis-Gerät und angeschlossenen peripheren Einrichtungen, speziell Sensoren bzw. Aktoren. Sie schließt den grundlegenden Gedanken ein, in der Signalleitung Mittel zur elektrisch-mechanischen Signalwandlung und in zweckmäßigen Ausführungen zugleich Mittel zur mechanoelektrischen Wandlung vorzusehen, um zunächst ein (normalerweise eingespeistes) elektrisches Wechselspannungssignal in eine mechanische Schwingung umzuwandeln und, bei der bevorzugten Ausführung, weiterhin eine Rück-Wandlung in ein elektrisches Signal zu bewerkstelligen. Diese Signalwandlung und, in einer weiteren zweckmäßigen Ausführung, das Vorsehen eines mechanischen Abschnitts im Signalweg erschließt Möglichkeiten zu den angestrebten Verbesserungen gegenüber herkömmlichen, rein elektrischen Signalleitungen, die nachfolgend in Verbindung mit Ausführungen der Erfindung skizziert werden.

Eine Ausführung der Erfindung sieht vor, dass dem mechanoelektrischen Wandler ein elektromechanischer Wandler vorgeschaltet ist derart, dass beide Wandler in Reihenschaltung einen Wechselspannungswandler bilden, und wobei auf einer Ausgangsseite des Wechselspannungswandlers ein höherohmiger Leitungsabschnitt als auf einer Eingangsseite vorgesehen ist. Dieser höherohmige Leitungsabschnitt ist weniger anfällig gegenüber elektromagnetischen Störungen (EMI) als etwa die in der bisherigen Schrittmachertechnik eingesetzten, relativ niederohmigen Leiter von Elektrodenleitungen. Das Vorsehen eines höherohmigen Leiters, bei vergleichbaren Leitungsverlusten, wird durch eine derartige Ausführung des elektromechanischen und des mechanoelektrischen Wandlers ermöglicht, dass am Ausgang des Wechselspannungswandlers ("Transformators") eine höhere Spannung in die Leitung eingespeist wird als auf der Eingangsseite geliefert wird.

Für eine Reihe wichtiger Anwendungen der vorgeschlagenen Anordnung ist es erforderlich, am Aktor eine elektrische Gleichspannung bereitzustellen. Für derartige Anwendungen ist dem mechanoelektrischen Wandler auf dessen elektrischer Seite bzw. dem Wechselspannungswandler auf dessen Ausgangsseite eine, insbesondere aktive, Gleichrichtereinrichtung nachgeschaltet.

In einer zweckmäßigen, kostengünstigen und vor allem Platz sparend realisierbaren Ausführung weist der oder mindestens ein mechanoelektrischer Wandler und/oder elektromechanischer Wandler ein Piezoelement auf. Miniatorisierte piezoelektrische Elemente sind in vielen Gebieten der Technik im ständigen Einsatz und haben einen hohen Entwicklungsstand erreicht, der ihre Nutzung zur Ausführung der Erfindung praktisch ohne wesentlichen zusätzlichen Entwicklungsaufwand erlaubt. Je nach konkreter Ausführung der Erfindung kann dabei auf vielgestaltige konstruktive Ausführungen einzelner oder in bestimmten seriellen oder parallelen Verschaltungsgruppen angebotener Piezoelemente zurückgegriffen werden.

In einer Ausgestaltung diese Ausführung ist vorgesehen, dass der Wechselspannungswandler ein auf zwei gegenüberliegenden Oberflächen und einer hierzu senkrecht stehenden Oberfläche mit Elektroden versehenes und als 3-Punkt-Transformator wirkendes Kombinations-Piezoelement aufweist. Bei einer weiteren zweckmäßigen Ausführung ist vorgesehen, dass der Wechselspannungswandler eine als 4-Punkt-Transformator wirkende Stapelanordnung von piezoelektrischen Elementen aufweist.

Mit mehreren mechanoelektrischen Wandlern zur unabhängigen Bereitstellung mehrerer Ausgangsspannungen ist eine weitergehende Ausschöpfung des Einsatzpotentials der Erfindung möglich. Wenn nämlich jedem mechanoelektrischen Wandler ein elektromechanischer Wandler vorgeschaltet ist derart, dass in der Signalleitung mehrere unabhängige Wechselspannungswandler zur parallelen Wandlung mehrerer eingespeister Eingangsspannungen in mehrere Ausgangsspannungen vorgesehen sind, lassen sich mehrere vom Basis-Gerät oder einer Sensorkonfiguration gelieferte Spannungen für die Übertragung auf dem elektrischen Leitungsabschnitt separat hochtransformieren.

Eine besonders vorteilhafte Ausgestaltung findet diese Ausführung der Erfindung darin, dass die mehreren Wechselspannungswandler mit einer gemeinsamen eingangsseitigen Zuleitung oder ausgangsseitigen Ableitung oder Elektrode verbunden sind und Mittel zur Frequenzkodierung der Ein- bzw. Ausgangsspannung vorgesehen sind. Hierdurch lässt sich nämlich die Anzahl der Leitungsdrähte in einer Elektrodenleitung oder einem ähnlichen Signalkabel wesentlich - im Idealfall bis auf einen einzelnen Leitungsdraht - reduzieren. Dies ermöglicht die Realisierung wesentlich dünnerer und flexiblerer und zudem erheblich kostengünstigerer Anschluss- bzw. Verbindungsleitungen.

Eine weitere Vereinfachung des Aufbaus insbesondere einer Elektrodenleitung eines Elektrostimulationsgerätes lässt sich erreichen, wenn die mehreren Wechselspannungswandler an jeweils einem ihrer Ausgänge mit einer gemeinsamen Bezugselektrode und an einem anderen ihrer Ausgänge mit einer separaten Elektrode verbunden sind. Hierdurch wird die Anzahl der Elektroden auf der Oberfläche der Elektrodenleitung verringert, und auch der konstruktive Aufwand für entsprechende Isolierungen wird reduziert.

Wie an einigen Punkten weiter oben bereits angemerkt, stellt eine Stimulations- und/oder Abfühlelektrodenleitung eines Elektrostimulationsgerätes, insbesondere eines Herzschrittmachers, implantierbaren Defibrillators oder Hörnerv-Stimulators, eine praktisch besonders wichtige Anwendung der Erfindung dar.

Unter Anordnungs-Aspekten ergibt sich neben den weiter oben erwähnten Vereinfachungs- und Kostensenkungsmöglichkeiten noch eine weitere: Hierbei ist mindestens ein äußerer Wandlungsabschnitt eines Gehäuses des Elektrostimulationsgerätes leitfähig und mit einem Eingang eines in der Signalleitung vorgesehenen Wechselspannungswandlers verbunden und an der Signalleitung, insbesondere an oder nahe einem distalen Ende derselben, ein mit einem Ausgang des in der Signalleitung vorgesehenen Wechselspannungswandlers verbundener Körperkontakt vorgesehen. Damit ist realisierbar, dass über das Gewebe eines Patienten ein Leitungsweg verläuft, der einen Leitungsdraht der Signalleitung ersetzt. Bei herkömmlichen Schrittmachern oder implantierbaren Defibrillatoren, die typischerweise in einem Titanblech-Gehäuse untergebracht sind, kann das gesamte Gehäuse den einen der Körperkontakte des Körper-Leitungsweges bilden. In einer hierzu alternativen Ausführung ist der leitfähige Abschnitt an einem nicht leitfähigen Gehäuse des Elektrostimulationsgerätes angebracht.

Weitere Vorteile und Zweckmäßigkeiten der Erfindung ergeben sich aus der nachfolgenden Beschreibung von Ausführungsbeispielen und -aspekten anhand der Figuren. Von diesen zeigen:
- Fig. 1: eine skizzenartige Darstellung einer ersten Ausführungsform der Erfindung,
- Fig. 2: eine skizzenartige Darstellung einer ersten Ausführung eines bei der Erfindung einzusetzenden Wechselspannungswandlers,
- Fig. 3: eine skizzenartige Darstellung einer zweiten Ausführung eines bei der Erfindung einsetzbaren Wechselspannungswandlers,
- Fig. 4: eine Darstellung einer weiteren Ausführungsform der Erfindung,
- Fig. 5: eine Darstellung einer noch weiteren Ausführungsform der Erfindung,
- Fig. 6: ein Prinzipschaltbild einer aktiven Gleichrichtereinrichtung, die im Rahmen der Erfindung eingesetzt werden kann,
- Fig. 7: eine Darstellung einer noch weiteren Ausführungsform der Erfindung,
- Fig. 8: eine Prinzipskizze einer zweckmäßigen Ausführung einer erfindungsgemäßen Anordnung mit einem Elektrostimulationsgerät.

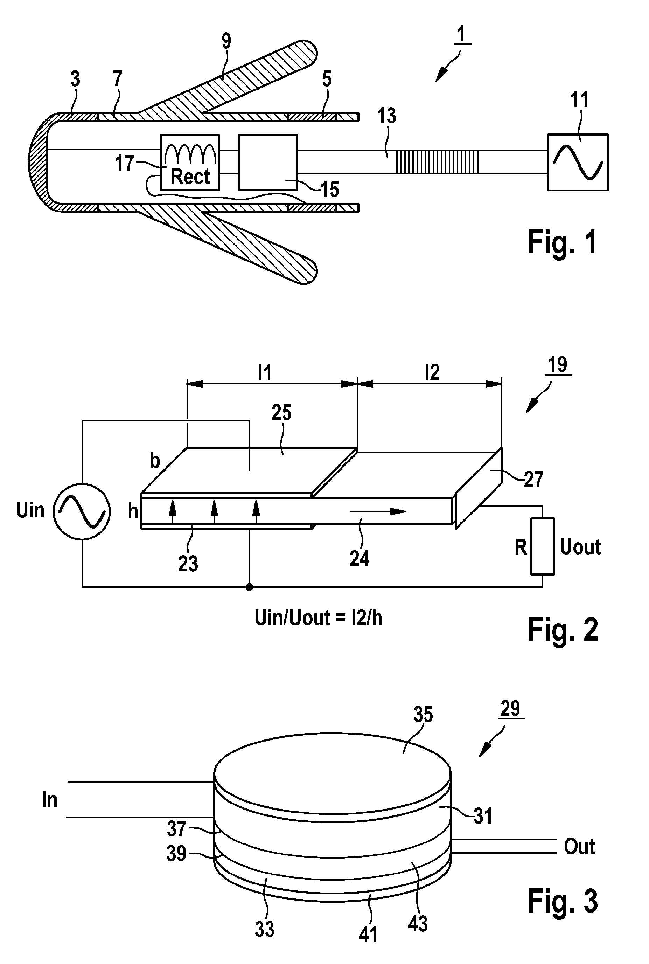

Fig. 1 zeigt schematisch das distale Ende einer neuartigen Schrittmacherelektrodenleitung 1 mit einer Tip-Elektrode 3 und einer Ringelektrode 5 in einem isolierenden Elektrodenkopf 7, von dem auch zwei Finnen 9 zur Verankerung im Trabekelwerk des Herzens gezeigt sind. Die Beaufschlagung der Elektroden mit Stimulationsimpulsen erfolgt hier über einen mechanischen Erreger 11, dem ein mechanischer Übertrager 13 und ein auf piezoelektrischer Basis arbeitender mechanoelektrischer Wandler 15 nachgeschaltet sind. Ausgangsseitig des mechanoelektrischen Wandlers wird eine der Schwingungsfrequenz des mechanischen Erregers entsprechende Wechselspannung bereitgestellt, welche durch eine Gleichrichtereinrichtung 17 in Gleichspannung umgewandelt und über zwei Ausgänge der letzteren der Tip-Elektrode 3 bzw. der Ringelektrode 5 zugeführt wird.

Fig. 2 zeigt schematisch einen im Rahmen der Erfindung als Wechselspannungswandler einsetzbaren piezoelektrischen "Transformator" 19, dessen Kernstück ein Kombinations-Piezoelement 21 ist, dessen Polarisationsrichtung und Beschaltung die Figur hinreichend klar zeigt. Das Kombinations-Piezoelement 21 mit einer Länge von (I1+I2), einer Breite b und einer Höhe h ist über einen Teil 21 seiner Hauptflächen mit einer ersten und zweiten Elektrode 23, 25 und an einer seiner kurzen Stirnseiten mit einer dritten Elektrode 27 versehen. An die erste und zweite Elektrode 23, 25 wird eine Eingangsspannung Uin angelegt, und über die dritte Elektrode 27 und einen Lastwiderstand R wird eine Ausgangsspannung Uout abgegriffen, deren Betrag bestimmt ist durch die Gleichung Uout/Uin=I2/h.

In Fig. 3 ist skizzenartig ein weiterer piezoelektrischer Wechselspannungswandler 29 gezeigt, der nach dem Prinzip des Vier-Punkt-Transformators arbeitet. Er umfasst zwei unterschiedlich dicke flach zylindrische bzw. scheibenförmige Piezoelemente 31, 33, deren Stirnflächen jeweils mit Anschlusselektroden 35, 37 bzw. 39, 41 belegt sind und zwischen denen eine Isolierscheibe 43 angeordnet ist. Wie auch beim vorgenannten, nach dem Drei-Punkt-Transformator-Prinzip arbeitenden piezoelektrischen Wandler 19 bestimmen die geometrischen Parameter der Piezo-Komponenten das Verhältnis Uin/Uout, also das Transformationsverhältnis. Üblicherweise wird die Ausgangsspannung (wie bei der Ausführung nach Fig. 1 beschrieben) vor ihrer Nutzung, etwa als Elektrostimulationsspannung, gleichgerichtet.

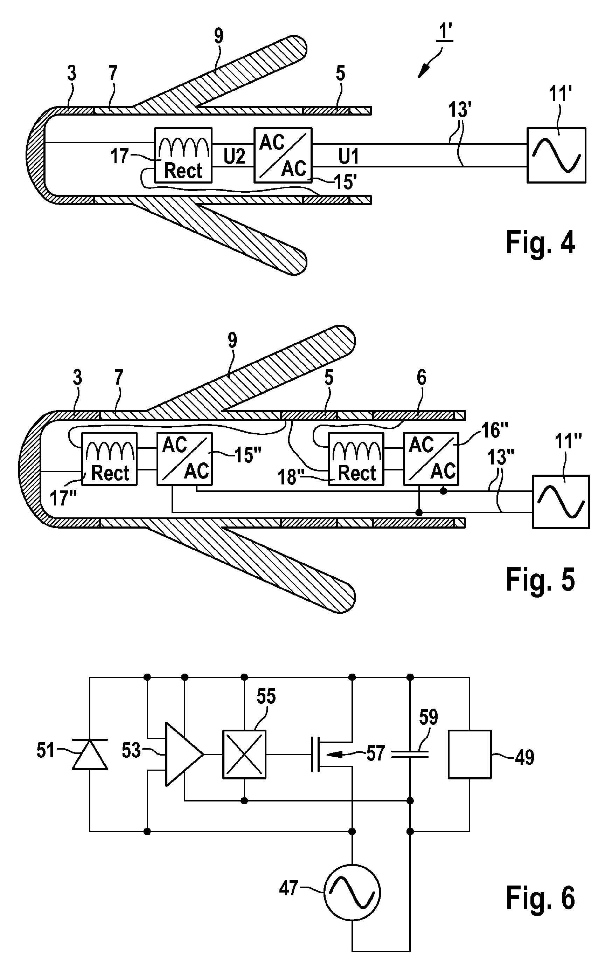

In Fig. 4 ist als zweite Ausführungsform der Erfindung eine Abwandlung der in Fig. 1 gezeigten ersten Ausführungsform dargestellt, wobei funktionsgleiche oder -ähnliche Teile mit den gleichen Bezugsziffern wie in Fig. 1 bezeichnet sind und hier nicht nochmals beschrieben werden. Die modifizierte Elektrodenleitung 1' unterscheidet sich von derjenigen aus Fig. 1 durch das Vorsehen eines piezoelektrischen Transformators 15- anstelle des mechanoelektrischen Wandlers 15 sowie durch eine elektrisch leitfähige, langgestreckte Verbindung (Zweidrahtleitung) 13' mit einem Impulsgenerator 11', der hier als elektrischer Generator ausgeführt ist.

Fig. 5 zeigt eine weitere erfindungsgemäße Elektrodenleitung 1", die sich als weitere Modifizierung der in Fig. 4 gezeigten Signalleitung ergibt. Der Elektrodenkopf trägt hier neben der Tip-Elektrode 3 und einer (ersten) Ringelektrode 5 noch eine zweite Ringelektrode 6, um separat zwei verschiedene Arten von Stimulationsimpulsen an das reizbare Gewebe eines Patienten übertragen zu können.

Angeschlossen ist die Elektrodenleitung 1" an einen elektrischen Generator 11", der zwei separate Ausgangsspannungen frequenzkodiert (d.h. auf unterschiedlichen Trägerfrequenzen aufgeprägt) bereitstellt. Beide Ausgangsspannungen des Generators 11" werden gemeinsam auf der Zweidrahtleitung 13' zum Elektrodenkopf übertragen und dort über zwei separate Transformator-Gleichrichter-Einheiten 15"/17" bzw. 16"/18" herunter transformiert und gleichgerichtet. Jeweils ein Ausgang beider Gleichrichtereinheiten 16" und 17" ist mit der hier als gemeinsame Bezugselektrode dienenden ersten Ringelektrode 5 verbunden, während der zweite Ausgang des Gleichrichters 17" mit der Tip-Elektrode 3 und der zweite Ausgang des Gleichrichters 18" mit der zweiten Ringelektrode 6 verbunden ist.

Die Ausführungen nach Fig. 4 und 5 bieten beide den Vorteil, dass in die jeweilige Elektrodenleitung eine höhere als die zur Stimulation des Gewebes angemessene Spannung eingespeist werden kann, weil durch die nahe dem distalen Leitungsende vorgesehenen Wechselspannungswandler eine Herunter-Transformation auf die benötigte Stimulationsspannung erfolgt. Dies ermöglicht nämlich den Einsatz höherohmiger Leitungsdrähte innerhalb der Elektrodenleitung, was wiederum eine Verringerung der Dicke und Steifigkeit und zudem eine Erhöhung der EMI-Störsicherheit bewirken kann. Die in Fig. 5 gezeigte Ausführung hat überdies den Vorteil, dass zur Fortleitung der verschiedenen Ausgangsspannungen des Stimulationsimpulsgenerators nur ein einziges Leitungspaar benötigt wird und der konstruktive Aufwand, die Dicke und die Steifigkeit gegenüber bekannten Elektrodenleitungen mit gleicher Funktion besonders deutlich reduziert werden kann.

Fig. 6 zeigt eine Prinzipskizze einer im Rahmen der Erfindung, nachgeschaltet zu einem Wechselspannungswandler, einsetzbaren aktiven Gleichrichtereinrichtung 45. Mit dem Symbol eines Wechselspannungsgenerators ist hier ein Wechselspannungswandler 47 symbolisch dargestellt, und die Schaltung umfasst zur Verbindung mit einer Last 49, etwa einer Stimulationselektrode, eine Diode 51, einen Spannungsvergleicher 53, einen Spannungsmultiplizierer 55, einen Schalttransistor 57 und einen Pufferkondensator 59 in der dargestellten Verschaltung. Der Wechselspannungsgenerator (AC/AC-Wandler) 47 erzeugt eine Wechselspannung in Flussrichtung der Diode 51.

Die Schaltung funktioniert folgendermaßen: Der Wechselspannungsgenerator erzeugt eine Wechselspannung in Flussrichtung der Diode 51. Bis zum Erreichen der Flussspannung baut sich eine Spannungsdifferenz über der Diode auf. Diese wird von dem Spannungsvergleicher 53 registriert, verstärkt und schaltet den Schalttransistor 57, der die Diode 51 überbrückt. Die Energie zum Versorgen der aktiven Elemente wird durch den Pufferkondensator 59 geliefert, der parallel zur Last betrieben wird. Sollte der Kondensator leer sein, erfolgt die Gleichrichtung zunächst passiv, bis der Kondensator genügend geladen ist, um den aktiven Teil der Schaltung zu speisen.

Durch die Nutzung mehrerer aktiver Schalter und Spannungsvergleicher kann ein Vollgleichrichter aufgebaut werden. Der Spannungsvergleicher kann auch ein Differenzierglied enthalten, um auch auf die Steilheit und nicht nur die Amplitude eines Signals zu reagieren.

In einer weiteren Abwandlung dieser aktiven Gleichrichtereinrichtung kann vorgesehen sein, dass der Spannungsvergleicher nicht hinter dem Wechselspannungswandler (Transformator), sondern vor diesem angeschlossen ist. Dies bietet den Vorteil, dass an dieser Stelle die Signalaussteuerung höher und die Anstiegsflanke des Signals steiler ist.

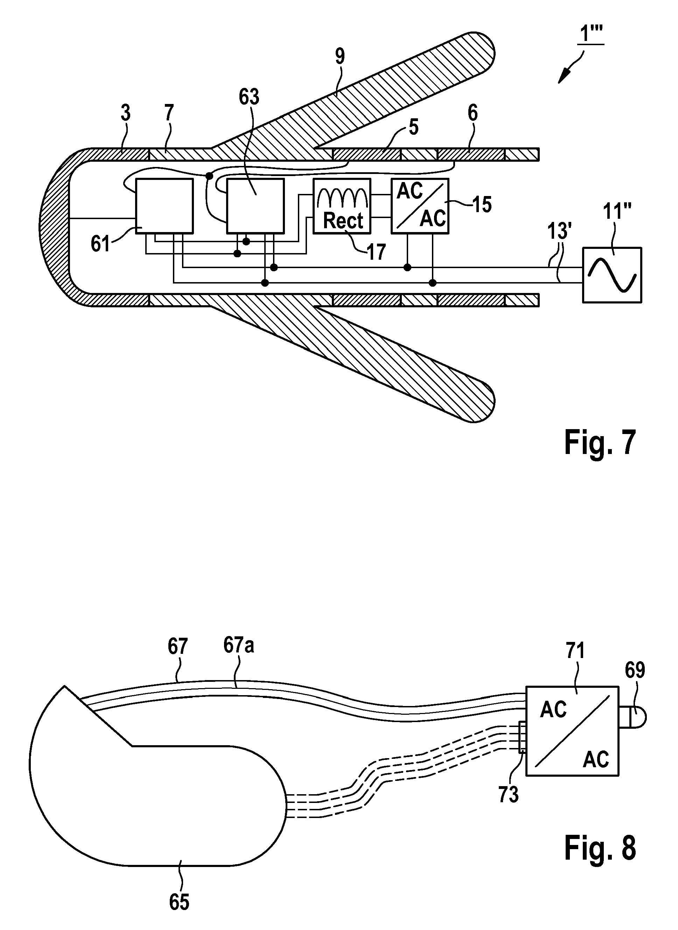

Fig. 7 zeigt als weitere Modifizierung der in Fig. 5 dargestellten und weiter oben beschriebenen Ausführung eine Elektrodenleitung 1"'. Die Bezugsziffern für gleiche oder gleichwirkende Teile sind hier wiederum die gleichen wie bei Fig. 5. Anstelle zweier Transformator-Gleichrichter-Kombinationen ist hier eine einzelne Transformator-Gleichrichter-Einheit 15/17 in Verbindung mit zwei frequenzgesteuerten Schaltern 61, 63 vorgesehen, die bei unterschiedlichen Frequenzen arbeiten und beide über die einzige Zweidrahtleitung 13' gespeist werden. Über die frequenzgesteuerten Schalter 61, 63 kann eine ausgewählte Anregungsspannung selektiv an die Elektrodenpaare 3/5 bzw. 3/6 angelegt werden.

Fig. 8 zeigt als Prinzipskizze eine Ausführung einer erfindungsgemäßen Anordnung aus einem Herzschrittmacher 65 (als Beispiel eines implantierbaren Basis-Gerätes), einer Elektrodenleitung 67 mit einer Stimulationselektrode 69 am distalen Ende und einem der Stimulationselektrode vorgeschalteten Wechselspannungswandler 71. Das Gehäuse des Schrittmachers 65 ist (wie üblich) aus Metallblech gefertigt und stellt somit einen ersten Körperkontakt der Gesamtanordnung dar, an den ein Pol des im Schrittmachergehäuse befindlichen, in der Figur nicht dargestellten (Wechselspannungs-)Generators angeschlossen ist.

Am Wechselspannungswandler 71 nahe dem Ende der Elektrodenleitung 67 ist ebenfalls ein Körperkontakt 73 vorgesehen, welcher mit einem Wandler-Eingang verbunden ist. Zwischen dem Schrittmachergehäuse und dem zweiten Körperkontakt 73 bildet sich ein "Körperleitungspfad" aus, der - zusammen mit einem Leitungsdraht 67a innerhalb der Elektrodenleitung 67 - einen Stromkreis zwischen dem Impulsgenerator und dem Wechselspannungswandler schließt.

Hierdurch kann ein Leitungsdraht der üblichen Zweidrahtleitung innerhalb der Elektrodenleitung eingespart werden. Insbesondere kann ein piezoelektrischer Transformator mit nur einem Leitungsdraht in der Elektrodenleitung angesteuert werden. Werden die Signale, wie weiter oben bereits erwähnt, frequenzkodiert übertragen, so können auch mehrere Nutzspannungen bzw. Kanäle über den einen Leitungsdraht in der Elektrodenleitung (und jeweils unter Nutzung des Körperleitungspfades) übertragen werden, und die Einsparung an Leitungsdrähten wird noch spürbarer.

Bei einem Basis- Gerät mit nicht leitfähigem Gehäuse können, in Abwandlung der letztgenannten Ausführung, auf dem Gehäuse leitfähige Abschnitte angebracht sein, die als geräteseitiger Körperkontakt dienen.

Die Ausführung der Erfindung ist nicht auf die oben erläuterten Beispiele und hervorgehobenen Aspekte beschränkt, sondern im Rahmen des Schutzbereiches der anhängenden Ansprüche auch in einer Vielzahl abgewandelter Ausführungen möglich.

## Patentansprüche

1. Signalleitung einer elektrischen Anordnung, insbesondere einer implantierbaren elektromedizinischen Anordnung, mit einem elektrischen Leitungsabschnitt oder Leitungsende und einem mechanoelektrischen Wandler zur Wandlung eines elektrischen Wechselspannungssignals in eine mechanische Schwingung.

2. Signalleitung nach Anspruch 1, wobei dem mechanoelektrischen Wandler ein elektromechanischer Wandler vorgeschaltet ist derart, dass beide Wandler in Reihenschaltung einen Wechselspannungswandler bilden, und wobei auf einer Ausgangsseite des Wechselspannungswandlers ein höherohmiger Leitungsabschnitt als auf einer Eingangsseite vorgesehen ist.

3. Signalleitung nach Anspruch 1 oder 2, wobei dem mechanoelektrischen Wandler auf dessen elektrischer Seite bzw. dem Wechselspannungswandler auf dessen Ausgangsseite eine, insbesondere aktive, Gleichrichtereinrichtung nachgeschaltet ist.

4. Signalleitung nach einem der vorangehenden Ansprüche, wobei der oder mindestens ein mechanoelektrischer Wandler und/oder elektromechanischer Wandler ein Piezoelement aufweist.

5. Signalleitung nach Anspruch 2 oder 3 und Anspruch 4, wobei der Wechselspannungswandler ein auf zwei gegenüberliegenden Oberflächen und einer hierzu senkrecht stehenden Oberfläche mit Elektroden versehenes und als 3-Punkt-Transformator wirkendes Kombinations-Piezoelement aufweist.

6. Signalleitung nach Anspruch 2 oder 3 oder 4, wobei der Wechselspannungswandler eine als 4-Punkt-Transformator wirkende Stapelanordnung von piezoelektrischen Elementen aufweist.

7. Signalleitung nach einem der vorangehenden Ansprüche, mit mehreren mechanoelektrischen Wandlern zur unabhängigen Bereitstellung mehrerer Ausgangsspannungen.

8. Signalleitung nach Anspruch 7, wobei jedem mechanoelektrischen Wandler ein elektromechanischer Wandler vorgeschaltet ist derart, dass in der Signalleitung mehrere unabhängige Wechselspannungswandler zur parallelen Wandlung mehrerer eingespeister Eingangsspannungen in mehrere Ausgangsspannungen vorgesehen sind.

9. Signalleitung nach Anspruch 8, wobei die mehreren Wechselspannungswandler mit einer gemeinsamen eingangsseitigen Zuleitung oder ausgangsseitigen Ableitung oder Elektrode verbunden sind und Mittel zur Frequenzkodierung der Ein- bzw. Ausgangsspannung vorgesehen sind.

10. Signalleitung nach Anspruch 8 oder 9, wobei die mehreren Wechselspannungswandler an jeweils einem ihrer Ausgänge mit einer gemeinsamen Bezugselektrode und an einem anderen ihrer Ausgänge mit einer separaten Elektrode verbunden sind.

11. Stimulations- und/oder Abfühlelektrodenleitung eines Elektrostimulationsgerätes, insbesondere eines Herzschrittmachers, implantierbaren Defibrillators oder Hörnerv-Stimulators.

12. Implantierbare Elektrostimulationsanordnung mit einem Elektrostimulationsgerät und einer Signalleitung nach einem der vorangehenden Ansprüche.

13. Anordnung nach Anspruch 12, wobei mindestens ein äußerer Wandlungsabschnitt eines Gehäuses des Elektrostimulationsgerätes leitfähig und mit einem Eingang eines in der Signalleitung vorgesehenen Wechselspannungswandlers verbunden ist und wobei an der Signalleitung, insbesondere an oder nahe einem distalen Ende derselben, ein mit einem Ausgang des in der Signalleitung vorgesehenen Wechselspannungswandlers verbundener Körperkontakt vorgesehen ist, derart, dass über das Gewebe eines Patienten ein Leitungsweg verläuft, der einen Leitungsdraht der Signalleitung ersetzt.

14. Anordnung nach Anspruch 13, wobei der leitfähige Abschnitt an einem nicht leitfähigen Gehäuse des Elektrostimulationsgerätes angebracht ist.
